# EUROPÄISCHE PATENTSCHRIFT

(11) **EP 1 833 529 B1**
(45) Veröffentlichungstag und Bekanntmachung des Hinweises auf die Patenterteilung: **20.02.2013**
(21) Anmeldenummer: 05850171.9
(22) Anmeldetag: 16.12.2005
(51) Int. Cl.: A61M 1/16

(54) **OXYGENATOR ZUM GASAUSTAUSCH**
OXYGENATOR FOR GAS EXCHANGE
OXYGENATEUR POUR L'ECHANGE GAZEUX

(30) Priorität: 21.12.2004 DE 102004062787; 13.05.2005 DE 102005023152
(43) Veröffentlichungstag der Anmeldung: 19.09.2007
(73) Patentinhaber: RWTH Aachen, 52062 Aachen (DE)
(72) Erfinder: CATTANEO, Giorgio, 76137 Karlsruhe (DE)
(74) Vertreter: Castell, Klaus
(86) Internationale Anmeldenummer: PCT/DE2005/002280
(87) Internationale Veröffentlichungsnummer: WO 2006/066553

(56) Entgegenhaltungen:
- WO-A-00/38816
- WO-A-94/03266
- WO-A-02/076530
- US-A- 6 106 776

## Beschreibung

Die Erfindung betrifft einen Oxygenator zum Gasaustausch.

Die extrakorporale Membranoxygenation (ECMO) ist eine invasive Therapie zum Aufrechterhalten der Körperfunktionen bei Lungenversagen. Sie beruht auf dem Austausch von Sauerstoff und Kohlendioxid mit dem Blut über semipermeable Membranen.

Traditionelle ECMO-Systeme bestehen aus einem Membranoxygenator, einem Wärmetauscher, einer Pumpe und diversen Schläuchen. Sie weisen ein großes Füllvolumen auf, haben jedoch aufgrund der gro-βen Blutkontaktoberflächen ein gesteigertes Hämolyse- und Thromboserisiko.

Die Forschung versucht seit geraumer Zeit, die erforderlichen Komponenten bestmöglich miteinander zu integrieren bzw. zu reduzieren, um eine bessere Hämokompatibilität zu erreichen. Mit einer Verringerung der Blutkontaktoberfläche im Gerät wird die Gefahr der Hämolyse und der Thrombenbildung verringert. Eine Reduzierung des Füllvolumens des Gerätes führt zusätzlich zu einer Verringerung der Hämodilution und der erforderlichen Zugabe von Blutprodukten. In den letzten Jahren sind zahlreichen neuartige Konzepte untersucht worden, bei denen teilweise sogar auf eine Pumpe zur Blutförderung verzichtet wird und dadurch die Komplexität des Systems, die Blutkontaktoberfläche, das Füllvolumen und die Blutschädigung reduziert wurden:

Makarewicz et al. (A Pumping Intravascular Artificial Lung with Active Mixing, ASAIO Journal 1993 ; 39:M466-M469) zeigen einen Oxygenator, bei welchem semipermeable Fasern schraubenförmig angeordnet sind und während des Einsatzes mit einer Drehzahl von bis zu 100 U/min um die Längsachse des Oxygenators rotieren. Ziel dieser Maßnahme ist eine bessere Durchmischung des Blutes, verbunden mit einer aktiven Blutförderung. Durch die Rotation wird das Blut nach dem Prinzip einer Archimedes'schen Schraube im Wesentlichen längs der Längsachse des Oxygenators nach vorne gefordert Dabei strömt es durch den schraubenförmigen Freikanal entlang der Hohlfasern. Ein solcher Oxygenator ist jedoch relativ komplex in der technischen Umsetzung. Zusätzlich sind die Fasern sehr kurz.

Fiore et al. (The Pumping Oxygenator: Design Criteria and First In-Vitro Results. Artif Organs. 2000 Oct; 24(10): 797-807) zeigen ein Oxygenatorsystem, welches mittels semipermeabler Membranfolien das Blut pulsatil fördert. Die Pulsatilität des Systems erfordert künstliche Klappen zum Einhalten der vorgegebenen Strömungsrichtung. Künstliche Klappen stellen jedoch eine zusätzliche kritische Stelle für die Thrombenbildung und die Blutschädigung dar.

Die US 6,106,776 A, die CA 2,286,091 A, die WO 98/46339 A1 und die US 6,348,175 B1 offenbaren Oxygenatoren, bei welchen semipermeable Fasern als Speicher eines Rades angeordnet sind. Bei einer Rotation wird das Blut durchmischt und der Gasaustausch erhöht. Auch dieses System ist jedoch relativ komplex aufgebaut und weist eine Vielzahl von Strörnungstotbereichen auf, in welchen das Risiko von Thrombenbildung oder Blutschädigung vorliegt. Außerdem kann das System nicht befriedigend dazu verwendet werden, das Blut bei gleichzeitigem Fördern des Bluts zuverlässig Gas zu tauschen: sobald die kombinierte Pump-Gastausch-Einheit in Rotation versetzt wird, strömt das Blut durch die einzelnen Kammern der Einheit. Dabei kann es zum einen einen relativ geraden, axial verlaufenden Weg durch die Scheiben wählen. Auf diesem Weg kommt es nur mit einem geringen Teilabschnitt der Hohlfasern in Kontakt. In den radial außen liegenden Bereichen der drei Kammern kommen die Fasern infolge des inhomogenen Strömungsbildes nur mit wenig Blutströmung in Kontakt. Zudem liegt ein Hauptströmungsweg des Bluts wohl dort, wo kein Strömungswiderstand in Form von Fasern vorliegt, nämlich in der außen liegenden Umgehung der rotierenden Scheiben.

Die WO 02/076530 A1 zeigt mehrere Ausführungsbeispiele von Oxygenatoren. Hierbei sind Hohlfasern kegelförmig angeordnet und werden fixiert und längs von Blut durchflossen.

Weitere Oxygenatoren zeigen die US 4,583,969, die EP 0 507 724 A1, die US 5,037,383, die US 5,814,01 und die WO 2004/016300 A1.

Die WO 94/03266 A1 offenbart einen Oxygenator zum Gasaustausch mit einem Bluteinlass und einem Blutauslass, wobei eine Hauptströmung des durch den Oxygenator gepumpten Blutes axial in den im Wesentlichen zylindrisch aufgebauten Oxygenator führt, dann umgelenkt wird und eine blutdurchlässige Gastauschwand mit gasdurchströmbaren Hohlfasern radial durchströmt. Die zylindermantelförmig stehende Wand aus gasdurchströmbaren Fasern wird von einem Motor angetrieben rotiert.

Die WO 00/38816 A1 offenbart einen Oxygenator mit einem Faserbündel, welches ebenfalls zylindermantelförmig angeordnet ist und gasdurchströmbar ist, um einen Gasaustausch mit dem vorbeiströmenden Blut zu ermöglichen.

In der US 6,106,776 wird ein Oxygenator offenbart, bei welchem gasdurchströmbare Hohlfasern zum Gasaustausch in mehreren parallelen Verteilerscheiben angeordnet sind, um die Gasaustauschoberfläche zu erhöhen.

Die WO 02/076530 A1 zeigt einen Oxygenator, bei welchem ein Faserbündel gasdurchströmbarer Hohlfasern zum Gasaustausch zunächst zum Einführen des Oxygenators in das Blutgefäß im Wesentlichen längs erstreckt liegt. Über eine Verschiebung eines Trägerelements wird eine axiale Verkürzung des Oxygenators innerhalb des Blutgefäßes erzwungen, so dass sich die zunächst im Wesentlichen längs erstreckten Fasern nach außen aufwölben und sich damit dem Blutströmungsweg in den Weg stellen. Das Blut wird dadurch gezwungen, durch die Fasern zu strömen, was den Gasaustausch verbessert.

Der vorliegenden Erfindung liegt die Aufgabe zugrundo, einen verbesserten Oxygenator zur Verfügung zu stellen.

Nach der Erfindung löst diese Aufgabe ein Oxygenator zum Gasaustausch mit den Merkmalen des Patentanspruchs 1.

Nach der vorliegenden Erfindung löst die Aufgabe ein Oxygenator zum Gasaustausch mit einem Bündel gasdurchströmbarer und blutumströmbarer Hohlfaser, welches in Rotation gleichzeitig den Gasaustausch und ein Pumpen des Bluts von einem Bluteinlass zu einem Blutauslass bewirkt, wobei in einer Gasführung von einem Gaseinlass zu einem Gasauslass eine Schaufel vorgesehen ist, die bei strömendem Gas das Faserbündel insbesondere rotatorisch antreibt.

Zunächst sei hierzu begrifflich erläutert, dass ein Oxygenator den Bluteinlass dort aufweist, wo das Blut in das Gehäuse des Oxygenators eintritt. Analog findet sich der Blutauslass dort, wo das Blut aus dem Gehäuse des Oxygenators wieder austritt.

Um das Blut mit Sauerstoff anzureichern, weist ein Oxygenator zugleich eine Gasführung auf. Als Gasführung sei der gesamte Leitungsweg für das Gas vom Gaseinlass zum Gasauslass verstanden. Analog zum Bluteinlass und Blutauslass wird unter dem Gaseinlass derjenige Anschluss des Oxygenatorgehäuses verstanden, wo Gas in die Gasführung eintreten soll, während als Gasauslass derjenige Anschluss des Gehäuses verstanden wird, wo das Gas wieder ausströmt. Vom Gaseinlass zum Gasauslass gibt es somit eine Gasführung. Die Gasführung führt durch die technische Kerneinheit des Oxygenators, nämlich diejenige Einheit, in welcher zwischen dem Blut und der Gasführung eine semipermeable Membran liegt, beispielsweise in Form einer Gastauschwand oder einer oder mehrer Hohlfasern.

Nach der vorgestellten Erfindung ist im Verlaufe des Gases zwischen dem Gaseinlass und dem Gasauslass eine ausgeprägte Schaufel vorgesehen. Eine Schaufel ist eine im Wesentlichen flächige Einrichtung, deren Oberfläche bei Vorbeiströmen von Gas durch die Gasführung ein Ungleichgewicht von Druckkräften aufweist, sodass sich eine resultierende Kraft ergibt. Diese Kraft kann aufgenommen und anderweitig genutzt werden, beispielsweise zum Antrieb des Faserbündels. Zu einem rotatorischen Antrieb eignet sich insbesondere ein Schaufelrad oder ein Flügelrad.

Die Erfindung ermöglicht auf besonders wirtschaftliche Weise den Antrieb des zum Bewegen vorgesehenen Faserbündels: zum Gasaustausch muss ohnehin Sauerstoff entlang der Gasführung gepumpt werden. Hiervon greift die Erfindung nach ihrem dritten Aspekt einen Teil der Strömungsenergie ab und nutzt diesen zum Antreiben des rotatorisch beweglichen Faserbündels. Eine weitere Energiequelle zum rotatorischen Antreiben des Faserbündels ist somit nicht notwendig.

Das Vorsehen einer Wand, welche so angeordnet ist, dass die Hauptströmung oder bevorzugt die gesamte Strömung des Blutes durch sie hindurch strömt, hat den großen Vorteil, dass das mit Sauerstoff anzureichernde Blut mit Sicherheit durch den hierzu vorgesehenen Mechanismus fließt und nicht die Möglichkeit hat, in einem großen Freikanal einfach nur daran vorbei zur strömen. Eine solche Wand bilden beispielsweise die Ausfächerungen der Hohlfasern in der WO 02/076530 A1 oder der WO 2004/016300 A1. Die ausgetächerten Hohlfaserbündel sind dicht aneinander gepackt und können daher als Wand bezeichnet werden. Dennoch sind die so gebildeten Wände blutdurchlässig.

Anders als die dort gezeigten Oxygenatoren ist die Wand kontinuierlich beweglich. Die kontinuierlich bewegliche Lagerung ermöglicht es, die Wand im Betrieb des Oxygenators entsprechend zu bewegen. Hierdurch wird nicht nur eine bessere Durchmischung des Blutes erreicht, sondern insbesondere im Vergleich zu pulsatilen Systemen die Gefahr der Blutschädigung erheblich verringert.

Gegenüber der US 6,106,776 A liegt dort wohl der Hauptströmungsweg für das Blut um die Scheiben herum vor, zumindest ist kein ausgeprägter Hauptweg für die Strömung erkennbar. Die hier bevorzugte Ausführung zwingt demgegenüber den bei weitem überwiegenden Teil des Blutes, wenn nicht sogar das gesamte Blut, eindeutig durch die Wand hindurch.

Die Gastauschwand kann besonders einfach und unter Ausnutzung bisheriger Forschungsergebnisse durch ein als Wand geformtes Hohlfaserbündel gebildet werden. Selbstverständlich können auch andere Strukturen zum Einsatz kommen, solange nur die Charakteristik einer Wand gegeben ist, also dass die Struktur dem Blut im Hauptströmungsweg durch den Oxygenator so in den Weg gestellt werden kann, dass sie diesen versperrt. Hierzu ist eine im Wesentlichen flächige Ausbildung der Wandstruktur vonnöten. Beispielsweise kann ein rechtwinkliges Raster von untereinander verbundenen Hohlfasern zum Einsatz kommen. Auch ist es nicht unbedingt notwendig, dass die Wand ausschließlich aus Gastauschelementen wie beispielsweise Hohlfasern gebildet ist. Generell wird jedoch der Wirkungsgrad des Gasaustausches besser sein, je höher der Anteil von gastauschfähigen Elementen an der gesamten Wandstruktur ist.

Die Gastauschwand ist um eine Rotationsachse rotierbar gelagert. Eine Rotation ist eine besonders ausfallsichere kontinuierliche Bewegung, die zudem gegenüber der ruhenden Struktur der Gastauschwand bei geeigneter Ausbildung keinen zusätzlichen Raum für die Rotation benötigt. Der Oxygenator kann daher kostengünstig und kompakt gehalten werden.

Es ist von besonderem Vorteil, wenn die Gastauschwand um die Rotationsachse herum parallel zu dieser liegt. Bei einer solchen Konfiguration dreht sich die Gastauschwand im Betrieb um die Rotationsachse. Dies ermöglicht es, durch geeigneten Aufbau des Oxygenators den Hauptströmungsweg des Blutes radial von innen nach außen zu richten. Dies gälte dann selbst ohne Rotation der Gastauschwand: Wegen des höheren Gasdurchflusswiderstands im Wandbereich, also beispielsweise im Faserbereich, strömt das Blut dort auf dem kürzesten Weg und demgemäß senkrecht durch die Gastauschwand hindurch. Im Innenbereich der Gastauschwand liegt ein kleinerer Strömungswider-stand vor, sodass sich das Blut dort axial bei konstantem Druck verteilen kann. Dies ermöglicht eine sehr definierte, homogene Blutströmung durch die Gastauschwand.

Im Gegensatz hierzu sind in der US 6,106,776 A die Strömungswege deutlich weniger definiert. Die Zentrifugalkraft wirkt hier entlang der Scheibe, die Fasern aufweist, wobei der kürzeste Weg für das Blut senkrecht zur Scheibe, parallel zur Rotationsachse ist. Im zentralen Bereich an der Welle können unter Umständen statische Wirbel und Blutstau entstehen.

Alternativ und kumulativ zum Vorgenannten löst nach einem Aspekt der Erfindung die Aufgabe auch ein Oxygenator zum Gasaustausch mit einem Bündel gasdurchströmbarer und blutumströmbarer Hohlfasern, wobei das Hohlfaserbundel in Rotation gleichzeitig den Gasaustausch und ein Pumpen des Bluts von einem Bluteinlass zu einem Blutauslass bewirkt und wobei das Faserbündel gestreckte Hohlfasern aufweist.

Es wurde bereits vorstehend erläutert, dass die Untersuchungen von Makarewicz et al. schraubenförmig angeordnete Hohlfasern verwenden, um gleichzeitig den Gasaustausch zu bewirken und das Blut durch den Oxygenator zu pumpen.

Die dort vorgestellten Fasern bilden infolge ihrer Kurze insgesamt eine recht kleine Oberfläche. Wenn sie verlängert würden, würde jede Faser für sich sehr instabil. Nach dem Liesigen Aspekt schlägt die vorliegende Erfindung insofern vor, ein Faserbündel mit gestreckten Hohlfasern zu verwenden, um den Gasaustausch und die Blutförderung zu bewirken. Bei einem Bündel gestreckter Hohlfasern ergibt sich der große Vorteil, dass diese unter Zugspannung eingebaut werden können, dies macht sie sehr stabil. Sie können dann ohne weiteres auch recht lang sein und somit eine große Gastauschoberfläche zur Verfügung stellen, ohne von der Blutströmung in unerwünschter Weise ausgelenkt zu werden oder unerwünschte großspaltige Durchlässe zu bilden.

Um ein besonders kompaktes Gehäuse zu erreichen, wird vorgeschlagen, dass das Faserbündel Hohlfasern aufweist, die zumindest im Wesentlichen in Längsrichtung des Oxygenators gestreckt sind. Während bei Hohlfasern, die abweichend von der Längsrichtung angeordnet sind, die maximale Faserlänge zwangsweise sehr beschränkt ist, kann eine Hohlfaser, welche parallel zur Längsachse des Oxygenators liegt, sich über nahezu dessen gesamte Länge erstrecken. Dies ermöglicht es, sehr große Gastauschflächen im Oxygenator vorzusehen.

Nach einem weiteren Aspekt wird Oxygenator mit einem eigenständigen pneumatischen Pumpenergievorrat und Pumpantrieb beschrieben.

Ein Oxygenator mit einem eigenen Pumpenergievorrat und einem eigenen Pumpantrieb ist autark. Insbesondere entfällt die Notwendigkeit, auf elektrischen Strom oder andere Energiequellen zuzugreifen. Ein solcher Oxygenator ist somit insbesondere zur Notfallbehandlung sehr gut einsetzbar.

Ein geeigneter eigenstäudiger Energievorrat und Pumpantrieb kann beispielsweise in einer Gaskartusche mit einem Druckminderer zur Verfügung gestellt werden. Gaskartuschen lassen sich auch in kleinen Bauformen kostengünstig herstellen und sind äußerst zuverlässig. Über den Druckminderer ausgangs der Gaskartusche oder zumindest im Verlaufe der Gasführung - wobei der Druckminderer nicht notwendigerweise ein eigenständiges Bauteil ohne übrige Funktion für den Gasaustausch und die Gasführung sein muss - sorgt dafür, dass das Gas nicht unkontrolliert schnell abfließt, sondern vielmehr über einen längerren Zeitraum mit gleichmäßiger Strömungsgeschwindigkeit genutzt werden kann.

Nach einem weiteren Aspekt wird ein Oxygenator zum Gasaustausch mit einem Bündel gasdurchströmbarer und blutumströmbarer Hohlfasern beschrieben, wobei das Faser-bündel in Rotation gleichzeitig den Gasaustausch und ein Pumpen des Bluts von einem Bluteinlass zu einem Blutauslass bewirkt, wobei das Faserbündel in Rotation gleichzeitig den Gasaustausch und ein Pumpen des Bluts von einem Bluteinlass zu einem Blutauslass bewirkt und wobei das Faserbündel Hohlfasern aufweist, die zumindest im Wesentlichen auf die Rotationsachse projizierbar sind.

Es wurde bereits erläutert, dass die Hohlfasern besonders kompakt rotierbar sind, wenn sie zumindest im Wesentlichen parallel zur Längsachse des Oxygenators angeordnet sind. Dem nun vorgestellten Aspekt liegt die Erkenntnis zugrunde, dass bei der Rotation des Faserbündels bei gleichzeitigem Durchströmen des Oxygenators mit Blut recht komplexe Strömungskräfte auf die Hohlfasern wirken können. Dabei besteht die Gefahr, dass die Hohlfasern eine unerwünschte und unvorhersagbare Formänderung einnehmen und schlimmstenfalls verknickten oder verkanten, sodass der Gasdurchfluss durch die Hohlfaser gefährdet oder zumindest vermindert ist. Um dies sicher zu vermeiden, wird vorgeschlagen, dass die Hohlfasern auf die Rotationsachse projizierbar sind, dass also bei einem unterstellten Blick von der Rotationsachse aus die Hohlfäsern der vorgeschlagenen Form als gerade Linien und parallel zueinender erscheinen. Dies gibt ein sehr geordnetes und äußerst stabiles Faserbild auch bei schneller Rotation der Fasern und bei Durchströmung mit Blut.

Ein weiterer Aspekt sieht einen Oxygenator zum Gasaustausch mit einem Bündel Hohlfasern vor, bei welchem das Faserbündel in Rotation gleichzeitig den Gasaustausch und ein Pumpen des Bluts von einem Bluteinlass zu einem Blutauslass bewirkt, wobei das Faserbündel durch seine Hohlfasern eine ausgeprägte Zentrifugalkraft auf das Blut ausübt, die zumindest im Wesentlichen senkrecht zu den Hohlfasern wirkt.

Eine solche Konstellation von Faserbündel und Blutströmungsbahn ist von besonderem Vorteil für eine gute Gastauschfunktion. Eine sehr gute Anströmung des Blutes zu den Fasern und zwischen diesen hindurch ist senkrecht zu diesen. Wenn die Fasern das Blut so zentrifugal beschleunigen, dass die Zentrifugalkraft senkrecht zu den Fasern steht, wird genau diese bevorzugte Anströmung forciert.

Dies ist insbesondere bei einer Blutführung von Vorteil, die bereits bei stehendem Faserbündel eine senkrechte Anströmung bewirkt, also beispielsweise wenn die Blutführung radial innerhalb des Faserbündels einen Ringspalt aufweist, innerhalb dessen sich zuströmendes Blut gleichmäßig verteilen und beruhigen kann, sodass sich axial eine homogene Druckverteilung einstellt.

Insbesondere kann das Faserbündel rotationssymmetrisch um die Rotationsachse des Faserbündels sein, womit Umwuchten bei der Rotation vermieden werden. Bei Einhalten der vorbeschriebenen Merkmale können die Fasern jeweils ohne Weiteres radial auch kurvig verlaufen, ohne an Stabilität des Faserbündels einzubüßen.

Eine bevorzugte Ausführungsform eines Oxygenators weist nach einem weiteren Aspekt zumindest etwa eine Zylindermantelform der Gastauschwand bzw. des Faserbündels auf. Eine zylindrische Form kann besonders gleichmäßig vom Blut durchströmt werden. Dabei können die einzelnen Fasern einer Faserbündelwand ohne weiteres verschiedene Konfigurationen haben, also beispielsweise nur eine axiale Erstreckung oder aber eine axial-tangentiale Erstreckung aufweisen. Letzterer Fall stellt sich unter anderem bei einem tordierten zylindrischen Faserbündel ein.

Insgesamt wird vorgeschlagen, dass ein ausgeprägter Freikanal für durch den Oxygenator gepumptes Blut vermieden ist. Sämtliche vorbekannten Oxygenatoren weisen zumindest einen ausgeprägten Freikanal für strömendes Blut auf. Im Falle der Vorschläge von Makarewicz et al. ist dies der schraubenförmige Kanal entlang der Mittelachse. Demgegenüber wird vorliegend vorgeschlagen, dass sich das Blut durch Gastauschelemente zwängen soll und diese damit bestmöglich umströmt, wobei sämtliches Blut unmittelbar an einem Gastauschelement entlang strömt. Dies verbessert die Gastauschergebnisse signifikant.

Wenn die Gastauschwand bzw. das Faserbündel die einzige Blutpumpeinrichtung des Oxygenators darstellt, kann der Bauraum für eine zusätzliche hierfür vorgesehene Vorrichtung eingespart werden. Insbesondere wird ein kleines Blutaumahmevolumen des gesamten Oxygenators geschaffen, was diesen nicht nur kostengünstiger herstellbar, sondern auch platzsparender und blutschonender gestalten lässt.

Ein Oxygenator nach demvorgestellten Erfindungsaspekt kann vorteilhaft so gestaltet sein, dass die Hohlfasern des Faserbündels eine zumindest im Wesentlichen homogene, dicht gepackte aber blutdurchlässige Faserwand bilden. Eine solche Wand mit zahlreichen Fasern als zumindest wesentliches Bauelement der Wand ermöglicht es, dass Blut durch sie hindurch strömen kann. Dabei sorgt der Aufbau der Wand dafür, dass eine Vielzahl von jeweils recht schmalen und mitunter stark verwundenen Pfaden von der Zuströmseite zur Abströmseite entsteht. Wenn Blut durch diese Spalten hindurchfließt, wird es stark durchmischt und garantiert in hohem Maße, dass sämtliches Blut direkt an einer Hohlfaser entlang fließt und dort den Gastausch vollziehen kann.

In einer devortugten Ansführungsform wird vorgeschlagen, dass der Oxygenator in einem Zulauf vom Bluteinlass zu der Gastauschwand bzw. zu dem Faserbündel einen Innenbereich in Gestalt eines längserstreckten Ringspaltes und/oder im Ablauf von der Gastauschwand bzw. vom Faserbündel zum Blutauslass einen Außenbereich in Gestalt eines längserstreckten Ringspaltes aufweist. Ein Ringspalt hat bei relativ kompakten Außenmaßen eine sehr große Oberfläche. Wenn das Blut in der Blutführung entweder vor oder hinter oder vor und hinter der Gastauschwand bzw. dem Faserbündel einen Ringspalt vorfindet, verteilt es sich auf eine große Oberfläche. Dabei kann das Gehäuse des Oxygenators kompakt gehalten werden. Eine solche Bauart ist besonders dann von Vorteil, wenn die Gastauschwand bzw. das Faserbündel konzentrisch mit dem Innenbereich und/oder dem Außenbereich ist und bezüglich des gemeinsamen Zentrums eine kontinuierliche Bewegung ausführt, insbesondere um das gemeinsame Zentrum rotiert.

Generell kennzeichnet sich ein Oxygenator nach der vorliegenden Erfindung dadurch, dass die Bewegung der Gastauschwand bzw. des Faserbündels eine ähnliche Bewegung von Blut entlang und/oder mit der Gastauschwand bzw. mit dem Faserbündel hervorruft, wobei diese Bewegung das Blut durch die Gastauschwand bzw. durch das Faserbündel treibt. Gedacht ist hier an eine Rotation. Wenn eine zylindrische Gastauschwand oder ein zylindrisches Faserbündel - wobei die Gastauschwand durch ein Faserbündel dargestellt sein kann - rotiert, während die Gastauschwand an ihrer radial nach innen gerichteten Seite Blut vorfindet, wird das Blut durch die Stokes'sche Haftung im unmittelbar an die Wand angrenzenden Bereich rotatorisch tangential angetrieben und mitgeführt. Die innere Reibung im Blut sorgt dafür, dass auch daran angrenzende Bereiche zur radial innen liegenden Seite der zylindrischen Anordnung in eine ebensolche Bewegung geraten. Dabei ruft die primär auf das Blut ausgeübte tangentiale Kraft eine kreisförmige Bewegung hervor, sodass zwischen dem Blut und der Gastauschwand zusätzlich eine Zentripetal- bzw. Zentrifugalkraft wirkt. Die Zentrifugalkraft des Bluts treibt dieses nach außen durch die rotierende Faserwand, genauer durch die dort vorhandenen schlitzartigen Kanäle, hindurch, ohne dass ein weiterer Antrieb vonnöten wäre.

Um etwaigen Druckschwankungen und/oder Ändenmgen in der Blutqualität vorzubeugen, wird vorgeschlagen, dass ein Oxygenator mit einem rotierenden Gastauschbauelement einen rotierenden Massekern aufweist, der mit dem Gastauschelement, also insbesondere mit der Gastauschwand bzw. mit dem Faserbündel, fest verbunden ist. Durch einen solchen Massekern, der über seine Massenträgheit eine Vergleichmäßigung des Verlaufs bewirkt, kann auch in hierfür vorgesehenen Kanälen die Gaszufuhr zum Faserbündel bzw. zur Gastauschwand und/oder die Gasabfuhr vorgesehen sein.

Insbesondere bei einer solchen Konstellation wird vorgeschlagen, dass ein Gaseinlass und ein Gasauslass gemeinsam bezüglich einer im Wesentlichen vom Bluteinlass zu Blutauslass definierten Längserstreckung des Oxygenatorgehäuses vor oder hinter einer Gastauscheinheit angeordnet sind. Die Anordnung von beiden Anschlüssen an einer Seite des Oxygenators, also insbesondere stromaufwärts oder stromabwärts des Gehäuses in Betrieb des Oxygenators, vereinfacht die Leitungsführung zum Oxygenator im Betrieb erheblich. Insbesondere kann ein einziger zweilumiger Katheter zum Anschluss an einen Doppelstutzen mit Gaseinlass und Gasauslass zum Einsatz kommen.

Um gerade bei einem rotatorischen Pumpprinzip im Oxygenator ein Energiemaximum abzugreifen, wird vorgeschlagen, dass ein Blutauslass exzentrisch an einem Außenbereich an der Gastauscheinheit angeordnet ist. Der Außenbereich ist derjenige Bereich, in welchen das Blut innerhalb des Oxygenatorgehäuses gepumpt wird, sodass dort die größte Energiehöhe vorliegt. Wenn der Blutauslass dort exzentrisch vorgesehen ist, insbesondere wenn er tangential angeschlossen ist, geht von dem rotatorisch strömenden Blut keine Energie durch unnötiges Umlenken verloren.

Wenn ein Sekundärkanal vom Bluteinlass zum Blutauslass unter Umgehung der Gastauschwand bzw. des Faserbündels führt, stellt sich dort eine Sekundärströmung im Betrieb des Oxygenators ein. Da zwischen dem Bluteinlass und dem Blutauslass im Oxygenator durch Pumpen die Energie des strömenden Blutes deutlich erhöht wird, ist an der zum Blutauslass gerichteten Seite des Sekundärkanals ein höherer Druck vorhanden als an der zum Bluteinlass gerichteten Seite. Insofern strömt das Blut durch den Sekundärkanal nicht nur in erheblich geringerem Maße als durch den Hauptströmungsweg, sondern zugleich auch in entgegengesetzter Richtung. Auf diese Weise kann die Sekundärströmung dazu genutzt werden, Strömungstotbereiche im Zulauf zur Gastauscheinheit auszuwaschen, ohne dass das strömende Blut die Gelegenheit hätte, in der Hauptströmungsrichtung durch den Sekundärkanal an der Gastauscheinheit vorbei durch den Oxygenator zu strömen. Das Maß der Sekundärströmung kann auf einfache Weise durch die Größe des Sekundärkanals definiert werden, also im Falle eines Ringspalts anhand der Spaltendicke.

Insbesondere um eine Gastauschwand mit oder aus einem Faserbündel besonders stabil auszuführen, können ein gaszufuhrseitiger Faserbündelanschluss und ein gasabfuhrseitiger Faserbündelanschluss über in dem und/oder neben dem Faserbündel liegende Stäbe verbunden sein. Der Vorteil von Stäben liegt darin, dass sie bei geeigneter Ausgestaltung sehr schmal sein können und somit das Strömungsbild nicht verfälschen. Insbesondere kann ein Stab in seiner Länge, seiner Ausrichtung und/oder in seinem Durchmesser recht ähnlich oder identisch mit den Hohlfasern vorgesehen sein. Auf diese Weise lassen sich stabilisierende Stäbe in und/oder neben dem Hohlfaserbündel anordnen, ohne dass mit einer Abweichung im Strömungsbild gegenüber reinem Hohlfaserbündeln ohne Verstärkung gerechnet werden muss.

In einem bevorzugten Ausführungsbeispiel werden die vorgenannten Stäbe als Teil der Gaszufuhr und/oder der Gasabfuhr genutzt. Eine weitere Kanalführung zwischen den unterschiedlichen Faserbündelanschlüssen ist somit nicht erforderlich.

Ein Oxygenator kann vorteilhaft ein Faserbündel einseitig hydrodynamisch lagern. Eine solche Lagerung empfiehlt sich besonders dann, wenn keine Stäbe zum Stabilisieren des Faserbündels eingesetzt sind. Der eigentlich recht lose Faseranschluss kann selbst in solch einem Falle durch die Rotation um sich herum ein Flüssigkeitspolster aus Blut aufbauen, sodass er nicht am Gehäuse anliegt.

Gemäβ der Erfindung ist es von Vorteil, wenn das Faserbündel dahingehend längs komprimiert ist, dass es eine Hohlfaser aufweist, die erheblich länger ist als zur geraden oder schraubenförmig tordierten Verbindung von gaszufuhrseitigem und gasabfuhrseitigen Anschluss erforderlich, sodass sie radial deutlich ausgeknickt ist und zwei Krümmungswendepunkte in ihrem Verlauf bezüglich der Rotationsachse aufweist. Eine solche Konstellation, die einem Regenschirm oder einer Haube ähnlich ist, schlägt auch die WO 02/076530 A1 vor. Bei Untersuchungen hat sich jedoch herausgestellt, dass in Verbindung mit den vorstehend beschriebenen Aspekten der hier vorliegenden Erfmdung überraschend hohe Gastauschwerte vorgefunden werden können. Insbesondere scheinen sich solche Werte einzustellen, wenn das Faserbündel kontinuierlich rotiert.

Um Blutstauungen zu verhindern, ist ein Oxygenator von Vorteil, bei welchem ein Gehäuse und/oder ein Massekern eine Spülbohrung aufweisen, die eine Verbindung zwischen einem im Betrieb des Oxygenators mit höherem Druck versehenen Bereich mit einem im Betrieb mit niedrigerem Druck versehenen Bereich direkt verbindet, sodass Blut mit hoher Geschwindigkeit vom Hochdruckbereich zum Minderdruckbereich fließt und dort eine etwa vorhandene Blutstauung ausspülen kann.

Infolge rotierender Elemente in der eigentlichen Gastauscheinheit und des hierdurch erzielten Zentrifugaleffekts ist es möglich, weniger dichte Stoffe als Blut in der Mitte der Rotation abzusaugen. Hier kann also insbesondere eingespültes oder eingebrachtes Gas abgesaugt werden. Nach einem weiteren bevorzugten Aspekt der Erfindund bewerkstelligt dies ein Oxygenator, bei welchem innerhalb einer sie umgebenden, im Betrieb rotierenden Gastauschwand zumindest etwa an einer Rotationsachse eine Gasabsaugeimichtung vorgesehen ist. Diese kann in eine ohnehin für das abzuführende Kohlendioxidgemisch vorhandene Gasabfuhr münden. Sie kann eine eigene Pumpe aufweisen, insbesondere eine Saug- oder Strahlpumpe.

Um eine solche Absaugwirkung zu erreichen, kann der Oxygenator Abfuhrschaufeln aufweisen, die bei Rotation im Betrieb des Oxygenators abströmendes Gas radial nach außen beschleunigen. Bei einer solchen Beschleunigung entsteht zum beschleunigten Gas ein Unterdruck, welcher direkt zum Absaugen eingesetzt werden kann.

Der Oxygenator ist besonders kompakt, wenn er genau eine Gastauschwand aufweist. Um den Wirkungsgrad zu erhöhen, können auch mehrere solche Einheiten vorgesehen sein. Dies können beispielsweise konzentrisch seriell, konzentrisch umeinander oder parallel zueinander liegen.

Während bei größeren Systemen ein separater Wärmetauscher erforderlich ist, ist bei der vorliegenden Erfindung bei einem kleinen Füllvolumen nicht notwendigerweise ein separater Wärmetauscher vorzusehen. Selbst wenn eine kleine Abkühlung erfolgen sollte, kann diese dadurch ausgeglichen werden, dass infolge der eingebrachten Energie und auftretender Reibung eine solche Abkühlung kompensiert werden kann.

Es sei ausdrücklich darauf hingewiesen, dass sich mit dem vorgestellten Aspekt der vorliegenden Erfindung alternativ und kumulativ zueinander auch vorteilhafte Dialysatoren und/oder Wärmetauscher herstellen lassen.

Die Erfindung wird nachfolgend anhand fünfter Ausführungsbeispiele und einer Versuchsbeschreibung unter Bezugnahme auf die Zeichnung näher erläutert. In der Zeichnung können funktional gleiche Bauelemente unterschiedlicher Oxygenatoren mit gleichen Bezugszeichen versehen sein. Es zeigen
- Figur 1: in einem schematischen Längsschnitt eine erste Ausführungsform eines Oxygenators,
- Figur 2: in einem ähnlichen Längsschnitt eine zweite Ausführungsform,
- Figur 3: in einem vergleichbaren Längsschnitt eine Ausführungsform eines erfindungsge mäßen Oxygenators,
- Figur 4: einen Querschnitt durch den Oxygenator in Figur 3 gemäß der dortigen Kennzeichnung IV-IV.
- Figur 5: eine schematische Darstellung einer alternativen Faserbündelkonfiguration in einem Längsschnitt,

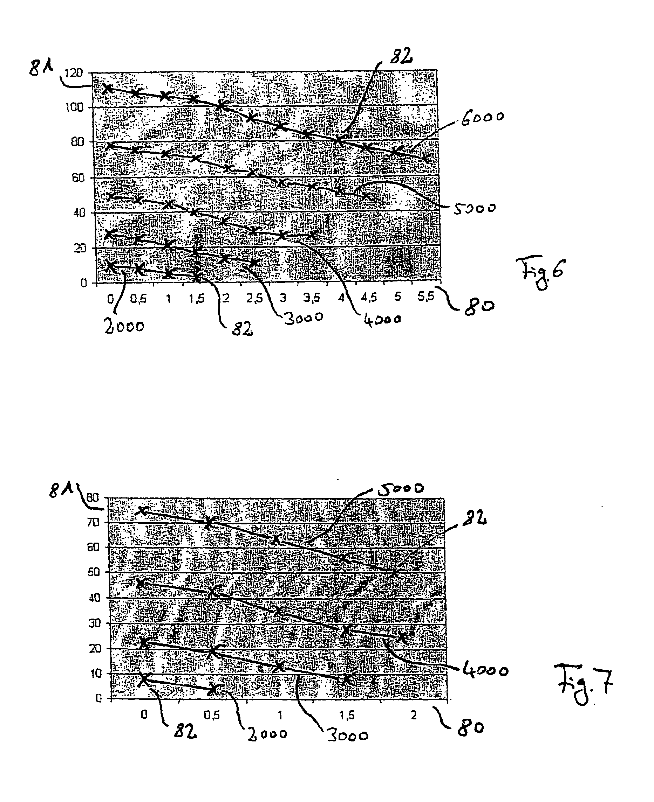

- Figur 6: eine erste grafische Darstellung von Messwerten zum Druckaufbau und zum Volumenstrom an einem Prototypen, wobei als strömendes Medium Wasser verwendet wurde,
- Figur 7: eine zweite grafische Darstellung von Messergebnissen, welche bei gleichem Versuchsaufbau, jedoch mit Blut als strömendem Medium ermittelt wurden,
- Figur 8: eine Veranschaulichung von Messergebnissen mit einem Versuchsaufbau mit einem gestülpten Faserbündel ähnlich der Form in Figur 5 und Wasser als strömendem Medium sowie
- Figur 9: eine Veranschaulichung von Messergebnissen mit dem gleichen Versuchsaufbau wie in Figur 8, jedoch mit Blut als strömendem Medium.

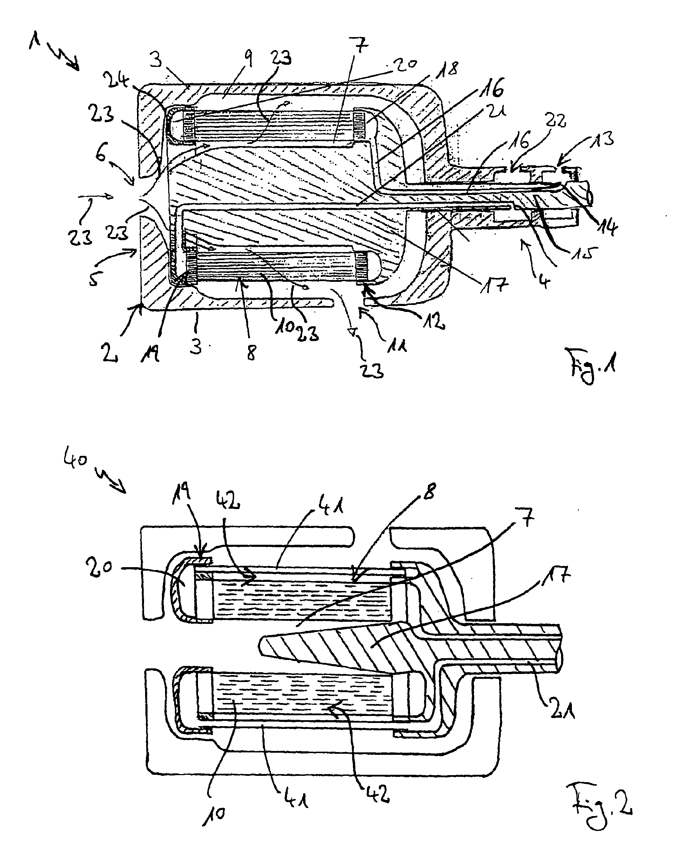

Der Oxygenator 1 in Figur 1 wird in seinem Außenumfang durch ein Gehäuse 2 definiert. Das Gehäuse 2 weist einen Korpus 3 und einen hieran fest angeformten Anschlusshals 4 auf. An einer im Betrieb stromaufwärts gerichteten Bodenfläche 5 weist der Oxygenator 1 einen zentralen Bluteinlass 6 auf. Von dem Bluteinlass 6 führt der Hauptströmungsweg durch den Oxygenator 1 zunächst in einen inneren Ringspalt 7. Um den zylindermantelförmigen inneren Ringspalt 7 schließt sich radial außen ein zylindrisches Faserbündel an. Radial außen am Faserbündel 8 befindet sich ein äußerer Ringspalt 9. Der Hauptströmungsweg für Blut, welches durch den Bluteinlass 6 in das Oxygenatorgehäuse 2 eintritt, führt somit vom Bluteinlass 6 in den inneren Ringspalt 7, von dort aus durch mäandrierende Schlitze zwischen Hohlfasern 10 (exemplarisch gekennzeichnet) hindurch in den äußeren Ringspalt 9 und von dort aus zu einem Blutauslass 11, welcher axial auf Höhe eines ersten, stromabwärts positionierten Faserbündelanschlusses 12 an einem Außenumfang des Gehäuses liegt, genauer knapp stromaufwärts des ersten Faseranschlusses 12, so dass derjenige Bereich der Hohlfasern 10, welcher unmittelbar neben dem ersten Faseranschluss 12 liegt, auf Höhe des Blutauslasses 11 ist.

Getrennt vom Strömungsweg für Blut weist der Oxygenator 1 eine Gasführung auf. Diese beginnt an einem Gaseinlass 13 an Anschlusshals 4. Mit dem Gaseinlass 13 ist eine korrespondierende Öffnung 14 in einer Welle 15 verbunden. Die Öffnung 14 führt zu einer Gaszuführbohrung 16 durch die Welle 15 und durch einen Teil eines rotierbar gelagerten Massekerns 17. Die Zuführbohrung 16 führt zu einem ersten Gasverteilungsring 18. An den ersten Gasverteilungsring 18 schließt sich das Faserbündel 8 in der Form eines Hohlzylinders an. Hierzu sind die semipermeablen Hohlfasern 10 an ihren beiden Enden zum ersten Faseranschluss 12 und zu einem zweiten Faseranschluss 19 zusammengegossen.

An den zweiten Faseranschluss 19 schließt sich eine zweite ringförmige Gasverteilung 20 an, die bei strömendem Gas streng genommen eher die Funktion einer Gassammelleitung hat. An diese schließt sich eine Gasabfuhrbohrung 21 an, die durch den Massekern 17 hindurch wiederum in die Welle 15 hinaus zum Anschlusshals 4 führt. Dort mündet die Abfuhrbohrung 21 in einen Gasauslass 22 direkt neben dem Gaseinlass 13.

Die Gasführung führt somit zwangsweise durch die Hohlfasern 10 des Faserbündels 8, und zwar in entgegengesetzter Richtung zum im Betrieb durch die Blutführung strömenden Blut am Faserbündel 8. Zur Verdeutlichung sind beispielhafte Strömungsbahnen für den Oxygenator 1 durchströmendes Blut beispielhaft eingezeichnet und mit der Bezugsziffer 23 gekennzeichnet.

Im Betrieb sorgt der Oxygenator 1 nicht nur für eine sehr gute Anreicherung des Bluts mit Sauerstoff, sondern er schafft es auch mit einem überraschend simplen System, das Blut durch die dann rotierenden Hohlfasern 11 des Faserbündels 8 zu pumpen: Das Blut im inneren Ringspalt 7 wird kreisförmig um den ebenfalls rotierenden Massekern 17 bewegt, wodurch es eine Zentrifugalkraft erfährt. Diese treibt es durch die Hohlfasern 10 in den äußeren Ringspalt 9, wo das Blut durch die Energieaufnahme an Druckhöhe gewonnen hat. Mit dem gesteigerten Druck verlässt es den Oxygenator 1 durch den Blutauslass 11. Da der Oxygenator 1 selbst für das Fördern des Bluts verantwortlich ist, ist keine weitere, herkömmliche Pumpe erforderlich.

Gleichzeitig führt die durch die Rotation des Faserbündels 8 hervorgerufene Blutdurchmischung zu einem erhöhten Gasaustausch. Dabei begegnet die technische Realisierung des Oxygenators 1 keiner bedeutenden Schwierigkeit, weil die Konfiguration der Hohlfasern 11 ähnlich wie bei herkömmlichen Oxygenatoren sein kann. Das gesamte System überrascht mit seiner Simplizität und vermeidet effektiv das Risiko einer Blutschädigung und Thrombenbildung.

Um das Faserbündel 8 mit dem Massekern 17 in Rotation zu versetzen, ist lediglich das Aufbringen eines Drehmoments auf die abgedichtete Welle vonnöten. Während das Blut im Betrieb vom inneren Ringspalt 7 zum äußeren Ringspalt 9 strömt, werden die Hohlfasern 10 zumindest in etwa senkrecht vom Blut angeströmt. Eine senkrechte Blutanströmung verursacht eine besonders ausgeprägte Blutdurchmischung, welche die günstigsten Voraussetzungen für einen effektiven Gasaustausch darstellt. Dabei erreicht das sauerstoffangereicherte Blut den äußeren Ringspalt 9 mit einer ausgeprägten rotatorischen Strömung. Diese nutzt der Blutauslass 11 zu einer blutschonenden Strömung 23 aus dem Gehäuse 2 hinaus, indem er zumindest in etwa tangential an das Gehäuse 2 und somit an den äußeren Ringspalt 9 angeschlossen ist.

Zwischen dem äußeren Ringspalt 9 und dem inneren Ringspalt 7 besteht neben dem Hauptströmungsweg 23 durch die Hohlfasern 10 noch eine kleine offene Passage in Form eines Sekundärkanals 24. Aufgrund des höheren Drucks im äußeren Ringspalt 9 fließt von dort im Betrieb des Oxygenators 1 ein kleines Maß an Blut durch den Sekundärkanal 24 zurück zur Zuströmung 23 vom Bluteinlass 6 zum inneren Ringspalt 7. Diese Rezirkulation um die Lagerstellen des rotierenden Massekerns 17 ist von Vorteil, weil eventuelle Strömungstotbereiche durchspült werden können und die damit verbundene Gefahr von Thrombenbildung reduziert wird.

Der Oxygenator 40 in Figur 2 zeigt gegenüber dem Oxygenator 1 aus Figur 1 eine konstruktive Alternative, bei welcher der zuströmende Sauerstoff durch kleine Rohre 41 in einem Außenbereich 42 des zylindrischen Faserbündels 8 geleitet wird. Diese Lösung ermöglicht eine besonders blutschonende Anströmung in den inneren Ringspalt 7 des Faserbündels 8, weil der dortige Raum nicht für Gasleitungen verwendet wird und weil der verkleinerte Massekern 17 ein vorteilhaftes Profil für die Blutanströmung aufweist.

Die kleinen Rohre 41 dienen im Betrieb des Oxygenators 40 zum einen zum Rückführen des Gases vom zweiten Faseranschluss 19, konkreter von der zweiten Gasverteilung 20, zurück zur Abführbohrung 21. Gleichzeitig stabilisieren sie das Faserbündel 8 und können zum konstruktiven Befestigen des Gassammelrings 20 benutzt werden.

Konstruktiv liegen zwischen den dargestellten Ausführungsformen der Oxygenatoren 1 und 40 in den Figuren 1 bzw. 2 mehrere weitere Möglichkeiten, bei welchen sich eine Gasleitung innerhalb des Faserbündels befände. Zusätzliche Varianten bestehen darin, dass das Gas durch einen mengenmäßigen Teil der Fasern 10 in eine Richtung und durch die übrigen Fasern 10 zurückströmt. In diesem Fall muss nicht notwendigerweise eine weitere Gasleitung parallel zu den Fasern vorgesehen werden. Konstruktiv mitunter nachteilhaft muss in diesem Falle jedoch eine Haltestruktur für das Faserbündel 8 an dem zweiten Faseranschluss 19 bereitgestellt werden.

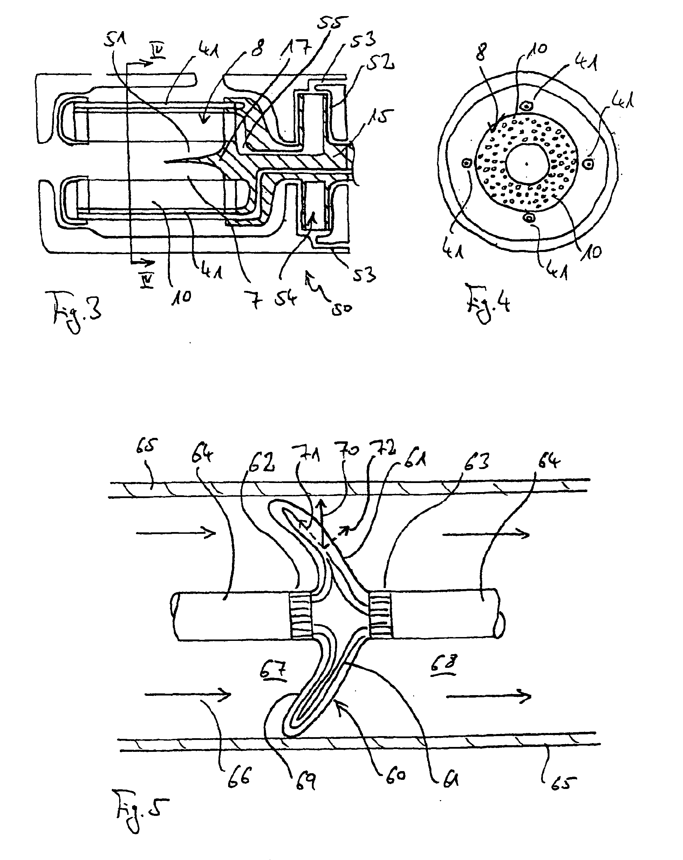

Der Oxygenator 50 in den Figuren 3 und 4 weist wie der Oxygenator 40 aus Figur 2 vier kleine Rohre 41 außerhalb des Faserbündels 8 auf. Im inneren Ringspalt 7 ist jedoch eine konkave Zuführung 51 zu den gestreckten Hohlfasern 10 (exemplarisch gekennzeichnet) am zentralen Massekern 17 vorgesehen, um das anströmende Blut möglichst rechtwinklig zu den zylindrisch angeordneten, dicht gepackten parallelen Hohlfasern 10 und durch diese hindurch zu führen.

Hierzu sei darauf hingewiesen, dass die rechtwinklige Zuführung zur Faserwand nicht vonnöten ist. Vielmehr bewirkt der vorteilhafte Aufbau von innerem Ringspalt 7, zylindrischer Faserwand 8 und äußerem Ringspalt 9 schon für sich genommen eine hervorragende Anströmung: im inneren Ringspalt 7 beruhigt sich das einströmende Blut. Die Druckverteilung stellt sich - abgesehen vom zentrifugalen Druckaufbau - weitgehend isotrop ein, insbesondere aber im axialen Verlauf praktisch konstant. Von dieser Ausgangslage strömt das Blut äußerst gleichmäßig durch die Faserwand 8 nach außen. Dort stellt sich im Ringspalt 9 wiederum eine leichte Vergleichmäßigung des Drucks im Blut ein, insbesondere im axialen Verlauf.

Axial stromabwärts der eigentlichen Gastauscheinheit weist die Gasführung des Oxygenators 50 eine kreisrunde Kammer 52 auf, in welche eine Gaszufuhrbohrung 53 mündet. Innerhalb der kreisrunden Kammer 52 liegt ein an die Welle 15 des Oxygenators 50 angeschlossenes oder angeformtes Schaufelrad 54. Über die Gaszufuhrbohrungen 53 zuströmendes Gas übt somit auf die Schaufeln des Schaufelrades 54 eine rotatorisch antreibende Kraft aus. Gleichzeitig wird das zuströmende Gas in die Schaufeln des Schaufelrades 54 aufgenommen und von dort über eine interne Zuführbohrung 55 zu den Hohlfasern 10 weitergeführt. Die Rückführung des Gases zum Gasauslass vom zweiten Faseranschluss 19 erfolgt wie bekannt durch die Welle 15.

Im Betrieb des Oxygenators 50 wird somit die Kraft für die Rotation des Massekerns 17 und somit auch des Faserbündels 8 von der Gasströmung selbst erzeugt. Bei einer solchen Variante ist somit kein externer oder anderweitiger Motor zum Antrieb des Faserbündels 8 erforderlich.

Der Oxygenator 50 kann beispielsweise mit seiner Gaszufuhrbohrung 53 an eine unter Druck stehende Sauerstoffkartusche angeschlossen werden, wobei die Sauerstoffkartusche oder der Oxygenator 50 selbst über einen Druckminderer verfügen sollte, um den Gasvolumenstrom über eine lange Zeit und möglichst konstant aufrecht erhalten zu können. Auf diese Weise ist das System tragbar und getrennt von jedem anderweitigen Gerät verwendbar, insbesondere ist es unabhängig von einer Sauerstoffflasche oder elektrischem Strom. Bei Notfällen wie beispielsweise Unfällen oder Vergiftungen kann der Oxygenator somit zum Einsatzort gebracht werden und dort autark arbeiten. Nach dem Gebrauch einer Sauerstoffkartusche kann eine neue Kartusche auf eine zügige und einfache Weise angeschlossen werden.

Es gilt allgemein, dass ein rotierender Hohlkörper, der aus einer durchlässigen Wandstruktur besteht, die umgebende Flüssigkeit vom inneren Bereich zum äußeren Bereich fördern kann. Bei dem entstehenden Strömungsbild durch die Wandstruktur hindurch stellt die Zentrifugalkraft die treibende Kraft dar. Wenn der rotierende Hohlkörper zusätzlich eine möglichst große Masse mit einer entsprechenden Trägheit aufweist und entsprechend zuverlässig Energie auf das Blut übertragen kann, wird die Durchmischung des Bluts infolge der Rotation noch begünstigt. Im bio-medizinischen Bereich lässt sich ein solches Konzept insbesondere für Oxygenatoren, also zur Sauerstoff- und Kohlendioxidübertragung, für Dialysatoren, also zur Stoffwechselproduktübertragung, und für Wärmetauscher einsetzen.

Dabei kann bei einem Gerät mit Hohlfasern deren Konfiguration verschiedenster Art sein: So besteht das Faserbündel 60 in Figur 5 zunächst aus flexiblen Hohlfasern 61 (exemplarisch gekennzeichnet), die an einem ersten Faseranschluss 62 und an einem zweiten Faseranschluss 63 zu einem Gasführungslumen 64 hin vergossen sind.

Die beiden Faseranschlüsse 62, 63 sind jedoch so nahe beieinander angeordnet, dass die Hohlfasern 61 in gestrecktem Zustand erheblich länger sind als die direkte Verbindung vom ersten Faseranschluss 62 zum zweiten Faseranschluss 63. Das Faserbündel 60 ist insofern komprimiert. Die Hohlfasern 61 knicken infolgedessen radial aus.

Da das Faserbündel 60 von einer zylindrischen Gehäusewand 65 umgeben ist und deren freier Durchmesser geringer ist als das Maß, um welches sich die ausgeknickten Hohlfasern 61 nach außen ausbreiten möchten, drückt die Gehäusewand 65 die Hohlfasern 61 radial nach innen. Wenn unter dem Reibschluss zwischen den Hohlfasern 61 und der Gehäusewand 65 zumindest der erste Faseranschluss 62 bezüglich einer Blutströmrichtung 66 stromabwärts bewegt wird, stülpen sich die ausgeknickten Hohlfasern 61 in eine Richtung, so dass sich die in Figur 5 dargestellte haubenartige Faserkonfiguration ergibt.

Wenn das Faserbündel 60 in dieser Konfiguration in Rotation versetzt wird, erfolgt wiederum eine Blutströmung von der konkaven stromaufwärtigen Seite 67 zur stromabwärtigen Seite 68 des Faserbündels 60. Auf diese Weise entsteht ein Volumenstrom, der von der Drehzahl des Bündels abhängt und den Strömungsweg des Bluts 66 durch das dichtgepackte, aber durchlässige Faserbündel 60 rührt.

Wie bei den Oxygenatoren 1, 40 und 50 aus den Figuren 1 bis 4 mit deren parallel zueinander und zur Längsachse des Oxygenators angeordneten Hohlfasern 10 beruht das Pumpphänomen auch beim Faserbündel 60 auf dem Entstehen einer zentrifugalen Kraft im Bereich der Fasern 61. In diesem Falle verläuft die Blutströmung durch die Hohlfasern 61 hindurch, jedoch nicht in derselben Richtung wie die Zentrifugalkraft wirkt: Dasjenige Blut, welches bei Rotationen des Faserbündels 60 zwischen den Fasern angeordnet ist oder durch Haftung von einer innersten Faserlage 69 mitgenommen wird, erfährt infolge der zwangsweisen Kreisbewegung eine Zentrifugalkraft 70. Die Zentrifugalkraft 70 kann in zwei Komponenten zerlegt werden, nämlich eine erste Komponente 71 entlang der Fasern und in eine zweite Komponente senkrecht zu den Fasern. Die zweite Kraftkomponente 72 fördert das Blut durch den Faserbereich hindurch in die stromabwärtige Seite 68. Die Beschleunigung von Blut entlang der Fasern durch die Kraftkomponente 71 wird durch Reibungskräfte erheblich reduziert, so dass die Beschleunigung infolge der zweiten Kraftkomponente 72 weit überwiegt.

Zum Untersuchen der Erfindung sind zwei Prototypen hergestellt worden, zum einen mit einer Faserbilndelkonfiguration im Wesentlichen wie beim Oxygenator 1 in Figur 1 sowie zum anderen im Wesentlichen wie beim Faserbündel 60 in Figur 5.

Im Versuchsaufbau zur zylindrischen Konfiguration ist das Faserbündel durch einen Käfig stabilisiert worden. Der Käfig weist außen der Hohlfasern Stäbe auf, so dass davon ausgegangen werden kann, dass der hydraulische Effekt des Käfigs äußerst ähnlich dem Vorsehen von Stabilisierungsrohren wie bei den Oxygenatoren 40, 50 in den Figuren 2, 3, und 4 ist. Die Gastauscheinheit wurde in einem festen Gehäuse angeordnet. Das feste Gehäuse ist zylindrisch ausgestaltet worden, wobei an einer Stirnseite zentral Flüssigkeit eingeführt wurde und auf axialer Höhe des Faserbündelendes ein Blutauslass in etwa tangential am Gehäuse angeschlossen wurde. Anschließend wurde das Faserbündel über eine abgedichtete Welle von einem Motor in Rotation versetzt. Zum Überprüfen des Pumpprinzips wurden stromaufwärts und stromabwärts des Faserbündels Druckmesssensoren angeordnet. Gleichzeitig wurde der Strömungsdurchsatz ermittelt. Als fließende und somit gepumpte Flüssigkeit wurden Wasser und Blut verwendet. Zum Darstellen der Ergebnisse wurde das rotierende Faserbündel auf verschiedene Umdrehungszahlen gebracht und der Druckaufbau infolge des rotierenden Faserbündels bei verschiedenen Volumenströmen ermittelt.

Bei dem zylindrischen Faserbündel ergeben sich mit Wasser als strömendem Medium die Kennwerte gemäß Darstellung 6 und mit Blut als strömendem Medium die Kennwerte gemäß Darstellung 7. Auf einer Abzisse 80 ist der Volumenstrom der jeweiligen Flüssigkeit in I/min angegeben. Auf einer Ordinate 81 findet sich der durch das rotierende Faserbündel erzeugte Druckaufbau in mmHg. Die Messwerte bei einer Umdrehungszahl des Faserbündels von 2000 U/min sind mit einzelnen Kreuzen 82 (exemplarisch gekennzeichnet) markiert. Sie sind zu einer Kennwertkurve 2000 verbunden. Analog sind die Kennwerte bei 3000 U/min zur Kennkurve 3000, die Messwerte bei 4000 U/min zur Kennkurve 4000, die Messwerte bei 5000 U/min zur Kennkurve 5000 und die Messwerte bei 6000 U/min zur Kennkurve 6000 verbunden.

Die Kennlinien, die mit Blut erfasst wurden, liegen niedriger als diejenigen Kennlinien, die mit Wasser erfasst wurden. Dies liegt wohl daran, dass das Faserbündel nicht nur die treibende Kraft, sondern auch gleichzeitig einen Widerstand für das Wasser bzw. das Blut darstellt. Der Widerstand hängt unter anderem von der Viskosität der jeweiligen Flüssigkeit ab. Die Viskosität liegt bei Blut höher als bei Wasser. Das Phänomen ist bei höheren Volumenstromwerten noch deutlicher. Die Kurve mit Blut fällt daher steiler ab.

Es muss jedoch darauf hingewiesen werden, dass die Messwerte lediglich zu einem ersten Prototypen erfasst wurden. Dieser weist naturgemäß keine optimale Auslegung hinsichtlich des Faserbündels auf. Beispielsweise würde ein längeres Bündel zu einer niedrigeren Blutgeschwindigkeit durch die Faser führen, was wiederum den Widerstand verringern würde. Der Durchmesser des Bündels und die Dichte der Fasern sind zwei zusätzliche Parameter, die zu einer Erhöhung der Leistungsfähigkeit des Bündels führen können.

Beim zweiten Versuch wurde das Bündel in die haubenartige Konfiguration wie in Figur 5 dargestellt gebracht. Um bereits durch das nicht rotierende Bündel einen Volumenstrom zu erzeugen, wurde eine zusätzliche Pumpe eingesetzt. Für den Volumenstrom bei stehendem Faserbündel wirkt dieses als reiner Widerstand.

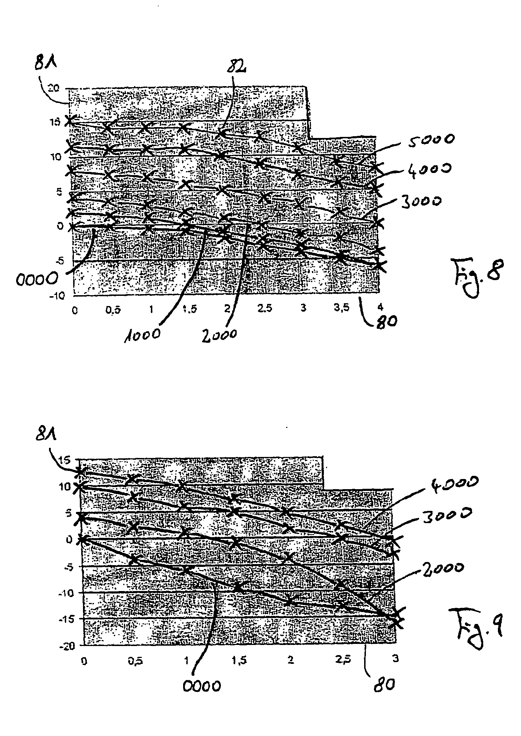

Wenn das Bündel in Rotation versetzt wird, ergibt sich eine zusätzliche Kraft, die ab einer bestimmten Drehzahl den Widerstand kompensiert. Die Messwerte 82 in den Figuren 8 und 9 für die Untersuchungen mit Wasser bzw. mit Blut zeigen diesen Effekt. In den Messwerten in den Figuren 8 und 9 sind diejenigen Messwerte 82, die bei stehendem Faserbündel ermittelt werden, mit der Kennziffer 0000 versehen.

Dabei sei noch darauf hingewiesen, dass das haubenförmige Faserbündel für den zweiten Prototypen zusätzlich zur umgestülpten Haubenform durch eine Verdrehung des ersten gegenüber dem zweiten Faserhalter leicht tordiert war.

### Bezugszeichenliste:

- 1: Oxygenator
- 2: Gehäuse
- 3: Korpus
- 4: Anschlusshals
- 5: Bodenfläche
- 6: Bluteinlass
- 7: innerer Ringspalt
- 8: Faserbündel
- 9: äußerer Ringspalt
- 10: Hohlfasern
- 11: Blutauslass
- 12: erster Faseranschluss
- 13: Gaseinlass
- 14: Öffnung
- 15: Welle
- 16: Zuführbohrung
- 17: Massekern
- 18: erster Gasverteilungsring
- 19: zweiter Faseranschluss
- 20: Gassammelring
- 21: Abführbohrung
- 22: Gasauslass
- 23: Strömungsbahn
- 24: Sekundärkanal
- 40: Oxygenator
- 41: Rohr
- 42: Außenbereich
- 50: Oxygenator
- 51: konkave Zuführung
- 52: kreisrunde Kammer
- 53: Gaszufuhrbohrung
- 54: Schaufelrad
- 55: interne Zuführbohrung
- 60: Faserbündel
- 61: Hohlfasern
- 62: erster Faseranschluss
- 63: zweiter Faseranschluss
- 64: Lumen
- 65: Gehäusewand
- 66: Blutströmungsrichtung
- 67: stromaufwärtige Seite
- 68: stromabwärtige Seite
- 69: innerste Faserlage
- 70: Zentrifugalkraft
- 71: erste Kraftkomponente
- 72: zweite Kraftkomponente
- 80: Abszisse
- 81: Ordinate
- 82: Messwertlareuz
- 0000: Drehzahl
- 1000: Drehzahl
- 2000: Drehzahl
- 3000: Drehzahl
- 4000: Drehzahl
- 5000: Drehzahl
- 6000: Drehzahl

## Patentansprüche

1. Oxygenator (1; 40: 50) zum Gasaustausch, mit einem Faserbündel (8: 60) gasdurchströmbarer und blutumströmbarer Hohlfasern (10: 61 das um eine Rotationsachse rotierbar gelagert ist, wobei das Faserbündel (8: 60) in Rotation gleichzeitig den Gasaustausch und ein Pumpen des Bluts von einem Bluteinlass (6) zu einem Blutawlass (11) bewirkt, ***dadurch gekennzeichnet, dass*** in einer Gasführung von einem Gaseinlass (13) zu einem Gasauslass (22) eine Schaufel vorgesehen ist, die bei strömendem Gas das Faserbündel (8; 60) rotatorisch antreibt.

2. Oxygenator nach Anspruch 1, ***dadurch gekennzeichnet, dass*** das Faserbündel (8: 60) als kontinuierlich bewegliche, blutdurchlässige Gastauschwand geformt ist, wobei ein Hauptströmungsweg durch den Oxygenator (1; 40: 50) gepumpten Blutes durch das Faserbündel (8; 60) und somit durch die Gastauschwand führt.

3. Oxygenator nach Anspruch 2, ***dadurch gekennzeichnet, dass*** das als Gastauschwand geformte Faserbündel (8 60) um die Rotationsachse herum parallel zu dieser liegt.

4. Oxygenator nach Anspruch 3, ***dadurch gekennzeichnet, dass*** das Faserbündel (8; 60) gestreckte Hohlfasern (10; 61) aufweist.

5. Oxygenator nach Anspruch 4, ***dadurch gekennzeichnet, dass*** das Faserbündel (8: 60) Hohlfasern (10) in Längsrichtung des Oxygenators (1; 40: 50) aufweist.

6. Oxygenator nach einem der Ansprüche 3 bis 5, ***dadurch gekennzeichnet, dass*** die Hohlfasern (10; 61) auf die Rotationsachse projizierbar sind.

7. Oxygenator nach Anspruch 6, ***dadurch gekennzeichnet, dass*** das Faserbündel (8; 60) rotationssymmetrisch um die Rotationsachse aufgebaut ist.

8. Oxygenator nach einem der vorhergehenden Ansprüche, ***dadurch gekennzeichnet dass*** das Faserbündel (8: 60) eine Zylindermantelform aufweist.

9. Oxygenator nach einem der vorhergehenden Ansprüche, ***dadurch gekennzeichnet, dass*** das Faserbündel (8; 60) die einzige Blutpumpeinrichtung des Oxygenators (1; 40: 50) darstellt.

10. Oxygenator nach einem der vorhergehenden Ansprüche, ***dadurch gekennzeichnet, dass*** der Oxygenator (1; 40: 50) in einem Zulauf vom Bluteinlass (6) zu dem Faserbündel (8: 60) einen Innenbereich in Gestalt eines längserstreckten inneren Ringspaltes (7) aufweist.

11. Oxygenator nach einem der vorhergehenden Ansprüche, ***dadurch gekennzeichnet, dass*** der Oxygenator (1; 40; 50) in einem Ablauf von dem Faserbündel zum Blutauslass (11) einen Außendbereich in Gestalt eines längserstreckten äußeren Ringspaltes (9) aufweist.

12. Oxygenator nach einem der vorhergehenden Ansprüche, ***dadurch gekennzeichnet, dass*** das Faserbündel (8: 60) primär eine tangentiale Kraft auf das Blut ausübt, woraufhin eine Bewegung des Blutes entsteht, so dass zwischen dem Blut und dem Faserbündel zusätzlich eine Zentrifugalkraft (70) wirkt.

13. Oxygenator nach Anspruch 12, ***dadurch gekennzeichnet, dass*** die Zentrifugalkraft (70) zumindest im Wesentlichen senkrecht auf dem Faserbündel (8: 60) steht.

14. Oxygenator nach einem der vorhergehenden Ansprüche, ***gekennzeichnet durch*** einen rotierenden Massekern (17), welcher mit dem Faserbündel (8: 60) fest verbunden ist.

15. Oxygenator nach einem der vorhergehenden Ansprüche, ***dadurch gekennzeichnet, dass*** ein Gaseinlass (13) und ein Gasauslass (22) gemeinsam bezüglich einer im Wesentlichen von Bluteinlass (6) zu Blutauslass (11) definierten Längserstreckung eines Oxygenatorgehäuses vor oder hinter einer Gastauscheinheit angeordnet sind.

16. Oxygenator nach einem der vorhergehenden Ansprüche, ***dadurch gekennzeichnet, dass*** ein Blutauslass (11) exzentrisch an einem Außenbereich an einer Gastauscheinheit angeordnet ist.

17. Oxygenator nach einem der vorhergehenden Ansprüche, ***gekennzeichnet durch*** einen Sekundärkanal (24), welcher vom Blutauslass (11) zum Bluteinlass (6) unter Umgehung des als Gastauschwand geformten Faserbündels (8: 60) führt.

18. Oxygenator nach einem der vorhergehenden Ansprüche, ***dadurch gekennzeichnet, dass*** ein gaszufuhrseitiger erster Faserunschluss (12) des Faserbündels (8: 60) und ein gasabfuhrseitiger zweiter Faseranschluss (19) des Faserbündels (8: 60) über im und/oder neben dem Faserbündel (8: 60) liegende Stäbe verbunden sind.

19. Oxygenator nach Anspruch 18, ***dadurch gekennzeichnet, dass*** die Stäbe als Rohre (41) geformt und Teil der Gasführung sind.

## Claims

1. Oxygenator (1: 40: 50) for gas exchange, with a fibre bundle (8: 60) of hollow fibres (10: 61) through which gas and around which blood can flow (10: 61), which is arranged in a rotating manner about an axis of rotation, wherein on rotation the fibre bundle (8: 60) simultaneously induces the gas exchange and pumping of the blood from a blood inlet (6) to a blood outlet (11), ***characterised in that*** in a gas flow system from a gas inlet (13) to a gas outlet (22) a vane is provided which drives the fibre bundle (8: 60) in a rotating manner when gas is flowing.

2. Oxygenator according to claim 1 ***characterised in that*** the fibre bundle (8: 60) is designed as a continuously moveable, blood-permeable gas exchange wall, wherein a principal flow path of blood pumped through the oxygenator (1: 40: 50) passes through the fibre bundle (8: 60) and thus through the gas exchange wall.

3. Oxygenator according to claim 2 ***characterised in that*** fibre bundle (8: 60) designed as the gas exchange wall is positioned around, and in parallel to, the axis of rotation.

4. Oxygenator according to claim 3 ***characterised in that*** the fibre bundle (8: 60) comprises stretched hollow fibres (10: 61).

5. Oxygenator according to claim 4 ***characterised in that*** the fibre bundle (8: 60) comprises hollow fibres (10) in the longitudinal direction of the oxygenator (1: 40: 50).

6. Oxygenator according to any one of claims 3 to 5 ***characterised in that*** the hollow fibres (10: 61) are projectable onto the axis of rotation.

7. Oxygenator according to claim 6 ***characterised in that*** the fibre bundle (8: 60) is built up in a rotation-symmetrical manner about the axis of rotation.

8. Oxygenator according to any one of the preceding claims ***characterised in that*** the fibre bundle (8: 60) is in the shape of a cylindrical casing.

9. Oxygenator according to any one of the preceding claims ***characterised in that*** the fibre bundle (8: 60) constitutes the only blood pumping device of the oxygenator (1: 40: 50).

10. Oxygenator according to any one of the preceding claims ***characterised in that*** in an intake line from the blood inlet (6) to the fibre bundle (8: 60), the oxygenator (1: 40: 50) has an interior in the form of an elongated inner annular gap (7).

11. Oxygenator according to any one of the preceding claims ***characterised in that*** in an outlet line from the fibre bundle to the blood outlet (11) the oxygenator (1: 40: 50) has an exterior in the form of an elongated outer annular gap (9).

12. Oxygenator according to any one of the preceding claims ***characterised in that*** the fibre bundle (8: 60) primarily exerts a tangential force on the blood, whereupon the blood is moved so that a centrifugal force (70) additionally acts between the blood and the fibre bundle.

13. Oxygenator according to claim 12 ***characterised in that*** the centrifugal force (70) is exerted at least essentially perpendicularly on the fibre bundle (8: 60).

14. Oxygenator according to any one of the preceding claims ***characterised* by** a rotating solid core (17) which is firmly connected to the fibre bundle (8: 60).

15. Oxygenator according to any one of the preceding claims ***characterised in that*** in relation to a longitudinal section of an oxygenator housing essentially defined from blood inlet (6) to blood outlet (11), a gas inlet (13) and a gas outlet (22) are jointly arranged before or behind a gas exchanger unit.

16. Oxygenator according to any one of the preceding claims ***characterised in that*** a blood outlet (1) is arranged eccentrically on an outer area on a gas exchange unit.

17. Oxygenator according to any one of the preceding claims ***characterised* by** a secondary channel (24) extending form the blood outlet (11) to the blood inlet (6) avoiding the fibre bundle (8: 60) designed as gas exchange wall.

18. Oxygenator according to any one of the preceding claims ***characterised in that*** first fibre connection (12) of the fibre bundle (8; 60) on the gas supply side and a second fibre connection (19) of the fibre bundle (8: 60) on the gas removal side are connected via rods located in and/or next to the fibre bundle (8: 60).

19. Oxygenator according to claim 18 ***characterised in that*** the rods are formed as pipes (41) and form part of the gas flow system.

## Revendications

1. Oxygénateur (1 : 40 : 50) pour l'échange gazeux comprenant un faisceau de fibres (8 : 60) de fibres creuses (10 ; 61) pouvant être traversées par le gaz et entourées par le sang, qui est disposé de manière rotative sur un axe de rotation, sachant que le faisceau de fibres (8 : 60) effectue en rotation à la fois l'échange gazeux et un pompage du sang d'une admission de sang (6) vers une sortie de sang (11), ***caractérisé en ce que*** dans une ligne de gaz d'une admission de gaz (13) vers une sortie de gaz (22), une aube est prévue qui entraine le faisceau de fibres (8 : 60) en rotation lorsque le gaz circule.

2. Oxygénateur selon la revendication 1, ***caractérisé en ce que*** le faisceau de fibres (8 : 60) est formé en tant que paroi d'échange gazeux laissant passer le sang et mobile en continu, sachant qu'un trajet de circulation principal à travers l'oxygénateur (1 : 40 : 50) conduit du sang pompé à travers le faisceau de fibres (8 : 60) et ainsi à travers la paroi d'échange de gaz.

3. Oxygénateur selon la revendication 2, ***caractérisé en ce que*** le faisceau de fibres (8 : 60) formé en tant que paroi d'échange gazeux est placé autour de l'axe de rotation et parallèlement à celui-ci.

4. Oxygénateur selon la revendication 3, ***caractérisé en ce que*** le faisceau de fibres (8 : 60) présente des fibres creuses (10 ; 61) étirées.

5. Oxygénateur selon la revendication 4, ***caractérisé en ce que*** le faisceau de fibres (8 : 60) présente des fibres creuses (10) en direction longitudinale de l'oxygénateur (1 : 40 : 50).

6. Oxygénateur selon l'une des revendications 3 à 5, ***caractérisé en ce que*** les fibres creuses (10 ; 61) peuvent être projetées sur l'axe de rotation.

7. Oxygénateur selon la revendication 6, ***caractérisé en ce que*** le faisceau de fibres (8 : 60) est monté en symétrie de rotation autour de l'axe de rotation.

8. Oxygénateur selon l'une des revendications précédentes, ***caractérisé en ce que*** le faisceau de fibres (8 : 60) présente une forme d'enveloppe cylindrique.

9. Oxygénateur selon l'une des revendications précédentes, ***caractérisé en ce que*** le faisceau de fibres (8 : 60) représente le seul dispositif de pompe à sang de l'oxygénateur (1 : 40 : 50).

10. Oxygénateur selon l'une des revendications précédentes, ***caractérisé en ce que*** l'oxygénateur (1 : 40 : 50) présente dans un système d'amenée de l'admission de sang (6) vers le faisceau de fibres (8 : 60), une zone intérieure sous la forme d'une fente annulaire (7) intérieure étirée en longueur.

11. Oxygénateur selon l'une des revendications précédentes, ***caractérisé en ce que*** l'oxygénateur (1 : 40 : 50) présente dans un système d'évacuation du faisceau de fibres vers la sortie de sang (11), une zone extérieure sous forme d'une fente annulaire (9) extérieure étirée en longueur.

12. Oxygénateur selon l'une des revendications précédentes, ***caractérisé en ce que*** le faisceau de fibres (8 : 60) exerce de manière primaire une force tangentielle sur le sang, sur quoi un mouvement du sang est produit de façon à ce qu'une force centrifuge (70) agisse en plus entre le sang et le faisceau de fibres.

13. Oxygénateur selon la revendication 12, ***caractérisé en ce que*** la force centrifuge (70) est au moins essentiellement verticale sur le faisceau de fibres (8 ; 60).

14. Oxygénateur selon l'une des revendications précédentes, ***caractérisé* par** un tore de ferrite (17) rotatif qui est relié fixement au faisceau de fibres (8 ; 60).

15. Oxygénateur selon l'une des revendications précédentes, ***caractérisé en ce* qu'**une admission de gaz (13) et une sortie de gaz (22) sont disposées avant ou après une unité d'échange de gaz conjointement par rapport à un étirement en longueur, essentiellement défini depuis l'admission de sang (6) vers la sortie de sang (11), d'un boitier d'oxygénateur.

16. Oxygénateur selon l'une des revendications précédentes, ***caractérisé en ce* qu'**une sortie de sang (11) est disposée de manière excentrique sur une zone extérieure sur une unité d'échange de gaz.

17. Oxygénateur selon l'une des revendications précédentes, ***caractérisé* par** un canal secondaire (24) qui conduit de la sortie de sang (11) à l'admission de sang (6) en contournant le faisceau de fibres (8 ; 60) formé en tant que paroi d'échange de gaz.

18. Oxygénateur selon l'une des revendications précédentes, ***caractérisé en ce qu'**un* premier raccordement de fibres (12) du faisceau de fibres (8 ; 60) côté amenée de gaz et un deuxième raccordement de fibres (19) du faisceau de fibres (8 ; 60) côté évacuation de gaz sont reliés via des tiges reposant dans le, et/ou près du, faisceau de fibres (8 ; 60).

19. Oxygénateur selon la revendication 18, ***caractérisé en ce que*** les tiges sont formées en tant que tubes (41) et font partie de la ligne de gaz.
